# EUROPEAN PATENT SPECIFICATION

(11) **EP 2 921 158 B1**
(45) Date of publication and mention of the grant of the patent: **05.04.2017**
(21) Application number: 13854575.1
(22) Date of filing: 08.11.2013
(51) Int. Cl.: A61Q 17/04, A61K 8/02, A61K 8/06, A61K 8/27, A61K 8/31, A61K 8/891

(54) **WATER-IN-OIL EMULSION SUNSCREEN COSMETIC**
SONNENSCHUTZ-KOSMETIKARTIKEL IN FORM EINER WASSER-IN-ÖL-EMULSION
CRÈME SOLAIRE DE TYPE ÉMULSION EAU DANS HUILE

(30) Priority: 13.11.2012 JP 2012249125
(43) Date of publication of application: 23.09.2015
(73) Proprietor: Shiseido Company Ltd., Chuo-ku Tokyo 104-0061 (JP)
(72) Inventor: NAGARE, Yuko, Yokohama-shi Kanagawa 224-8558 (JP); YAMAGUCHI, Kazuhiro, Yokohama-shi Kanagawa 224-8558 (JP)
(74) Representative: Ter Meer Steinmeister & Partner
(86) International application number: PCT/JP2013/080194
(87) International publication number: WO 2014/077189

(56) References cited:
- EP-A1- 1 550 429
- EP-A1- 2 149 361
- EP-A1- 2 505 182
- WO-A1-2012/026235
- WO-A1-2013/031374
- WO-A1-2013/108515
- JP-A- H09 255 544
- JP-A- 2001 058 934
- JP-A- 2010 184 885
- US-A1- 2007 264 292
- US-A1- 2010 233 103
- US-B1- 6 534 044

## Description

### Technical Field

The present invention relates to a sunscreen cosmetic. In more detail, the present invention relates to a water-in-oil emulsion sunscreen cosmetic from which unpleasant odor specific to sunscreen cosmetics is unperceivable despite high capability of blocking ultraviolet light and that is smoothly and well spreadable without feeling coarse and, in addition, not sticky after applied.

### Background of the Invention

Zinc oxide powder is formulated in many sunscreen cosmetics in order to block a long wavelength ultraviolet light. Zinc oxide is, however, difficult to be stabilized during dispersing, and even in some cases, the catalytic activity of zinc oxide *per se* may impair the stability of the formulation system. Considering maintaining function of the ultraviolet light scattering agent and improving stability and texture of the formulation system, the surface of zinc oxide has been hydrophobized with e.g. a silicone, a metallic soap, a fatty acid, a fluorine compound.

However, zinc oxide hydrophobized with an alkylalkoxysilane, widely used as a hydrophobizing agent, has specific odor and accordingly some users perceive the odor *per se* specific to sunscreen cosmetics and feel unpleasant. In the light of this fact, use of modified powder of which base powder, e.g. zinc oxide, has a blocking function against ultraviolet light and of which surface is coated with a silicone compound having a specific structure, has been proposed for the application (Patent Document 1.)

Meanwhile, an attempt has also been made to maintain the time-course stability of formulations by coating the surface of zinc oxide with silica (silicic anhydride), by which an elution of zinc ion or catalytic activity of zinc ions may be inhibited.

For example, Patent Document 2 discloses that a reaction between zinc oxide and a free fatty acid in the system can be inhibited and thereby the stability can be improved by formulating a composite powder of which microparticle of zinc oxide 0.1 µm or less in size is coated with silicic anhydride providing 5 % by weight or more and 30 % by weight or less of the coating ratio to zinc oxide and further processed the surface thereof with silicone providing 3 % by weight or more and 12 % by weight or less of coating ratio, and one or more than two polyoxyalkylene-modified polysiloxanes.

Also, Patent Document 3 discloses an external skin agent containing a benzotriazole derivative and silica-coated metal oxide. The use of silica-coated metal oxide suppresses discoloration of the agent and improves the long-lasting efficiency on blocking ultraviolet light.

However, in some cases, it is problematic that the cosmetic formulated with silica-coated powder is lacking of smoothness and has poor spreadability, and cause coarseness when they are applied to the skin.

### Prior Arts

### Patent Document

Patent Document 1: JP2010-270073 A
Patent Document 2: JP4011799 B
Patent Document 3: JP2009-114088 A

### SUMMARY OF THE INVENTION

### Problems to be solved

An object of the present invention is to provide a sunscreen cosmetic from which unpleasant odor specific to zinc oxide is unperceivable and that can provide smoothly and well spreadable application-sensation without feeling coarse and stickiness even after applied.

### Means to solve the problems

The inventors of the present invention studied intensively to solve the above problems and consequently found that an unpleasant odor specific to zinc oxide could be suppressed by using hydrophobized silica-coated microparticle of zinc oxide. And further, the inventors found that coarseness due to the formulated hydrophobized silica-coated microparticle of zinc oxide was suppressed and smoothly and well spreadable excellent texture was brought in reality by formulating a combination of the hydrophobized silica-coated microparticle of zinc oxide and a predetermined amount of volatile hydrocarbon oil and volatile dimethicone, and the present invention has been completed, accordingly.

Specifically, the present invention provides a water-in-oil emulsion sunscreen cosmetic comprising; (a) a hydrophobized silica-coated microparticle of zinc oxide, (b) a volatile hydrocarbon oil, (c) a volatile dimethicone, and (d) optionally a semi-solid oil formulated in an amount of 3% by mass or less on the basis of the total amount of the sunscreen cosmetic, wherein the total amount of (b) the volatile hydrocarbon oil and (c) the volatile dimethicone is in the range of 3 to 45% by mass on the basis of the total amount of the sunscreen cosmetic.

### Effects of the Invention

According to the present invention, a sunscreen cosmetic suppresses unpleasant odor specific to zinc oxide by using (a) a hydrophobized silica-coated microparticle of zinc oxide in addition to providing an excellent preventive effect on sunburn. Further, by formulating a combination of (a) the hydrophobized silica-coated microparticle of zinc oxide and a predetermined amount of (b) a volatile hydrocarbon oil and (c) a volatile dimethicone, problematic worsening of texture due to formulation of (a) the hydrophobized silica-coated microparticle of zinc oxide can be suppressed.

### Description of Embodiments

Hereafter, the inventor further describes the present invention in detail.

### < (a) Hydrophobized silica-coated microparticle of zinc oxide>

According to the present invention, (a) a hydrophobized silica-coated microparticle of zinc oxide (or hereafter simply "component a" in some cases) formulated in the sunscreen cosmetic is a microparticle obtained by processing the surface of a composite powder (silica coated zinc oxide) with a hydrophobizing agent following uniformly coating silica on the surface of a microparticle of zinc oxide to obtain the composite powder.

The average particle diameter of the microparticle of zinc oxide is preferably in the range of 0.01 to 0.1 µm, more preferably in the range of 0.01 to 0.05 µm. The commercially available microparticle of zinc oxide may include FINEX-25, FINEX-50, and FINEX-75 (all from Sakai Chemical Industry Co., Ltd.), ZnO350 (Sumitomo Osaka Cement Co., Ltd.), ZINCOX SUPER-10, ZINCOX SUPER-20R, and ZINCOX SUPER-30 (all from Hakusuitech Co., Ltd.), and Z-COTE (Sun Smart, Inc.)

Silica-coated zinc oxide can be obtained by forming a silica-coated layer on the surface of the above microparticle of zinc oxide using a well-known method. Specifically, for example, silica-coated zinc oxide can be obtained by adding sodium silicate to an aqueous solution of the microparticle of zinc oxide to neutralize followed by uniformly coating silicic anhydride on the surface of powdery particle of the above microparticle of zinc oxide under a highly dispersed condition maintained using e.g. a sand grinder mill. Further, silica-coated zinc oxide can also be obtained by adding alkoxysilane to uniformly coat silicic anhydride on the surface of powdery particle of the above microparticle of zinc oxide following highly dispersing the microparticle of zinc oxide in an organic solvent. Any other methods than the above methods can be used as long as a predetermined amount of silica can be coated at the end.

The silica coating of the microparticle of zinc oxide is preferably in the range of 5 to 30% by mass and particularly preferable in the range of 10 to 23% by mass on the basis of the total silica-coated zinc oxide. When the silica coating is less than 5% by mass, it is difficult to uniformly coat the periphery of the microparticle of zinc oxide, while when the silica coating is over 30% by mass, worsening of feeling-in-use, e.g. squeakiness due to silica, takes place.

The surface of the above obtained silica-coated zinc oxide is further processed with a hydrophobizing agent to obtain the hydrophobized silica-coated microparticle of zinc oxide usable in the present invention.

The hydrophobizing agent may include known agents to date but not particularly limited. Specifically, a silicone compound is preferable, e.g. a kind of silicone oil including dimethyl polysiloxane (dimethicone), methyl phenyl polysiloxane, and methyl hydrogen polysiloxane, a kind of alkylsilane including methyltrimethoxysilane, ethyltrimethoxysilane, hexyltrimethoxysilane, and octyltrimethoxysilane, and a kind of fluoroalkylsilane inclduing trifluoromethyl ethyl trimethoxysilane and heptadecafluorodecyl trimethoxysilane. One or a mixture of more than two of them can be used.

The method for hydrophobizing may include a known method to date but not particularly limited. Further, the silica-coated zinc oxide is not limited to silica coated zinc oxide fully-coated with the hydrophobizing agent as long as hydrophobicity due to hydrophobizing is assured. Hydrophobicity thereof may be assured using a known method.

As described above, the hydrophobized silica-coated microparticle of zinc oxide usable in the present invention has a particle layer consisting of silica between the layer of hydrophobizing agent and the microparticle of zinc oxide. It is presumed that the unpleasant odor specific to zinc oxide can be suppressed because the in-between particle layer thereof under the outermost layer seals off the source of the odor.

Further, a commercially available hydrophobized silica-coated microparticle of zinc oxide may be used. The commercially available agent may include FINEX-30W-LP2 and FINEX-50W-LP2 (both from Sakai Chemical Industry Co., Ltd.) and Maxlight® ZS-032-D (Showa Denko Co., Ltd.)

A hydrophobized silica-coated microparticle of zinc oxide can be appropriately formulated in accordance with the desired SPF value and the formulation amount of hydrophobized silica-coated microparticle of zinc oxide is not particularly limited, but preferably in the range of 0.1 to 20% by mass, more preferably in the range of 1 to 15% by mass, and most preferably in the range of 3 to 10% by mass on the basis of the total amount of the sunscreen cosmetic. When the formulation amount is less than 0.1% by mass, the effectiveness as a sunscreen may be insufficient, and on the other hand when the amount thereof is more than 20% by mass, it becomes undesirable because a coarse feeling or a visually excessive whiteness likely takes place.

### <(b) Volatile hydrocarbon oil>

According to the present invention, (b) a volatile hydrocarbon oil (or hereafter simply "component b" in some cases) formulated in the sunscreen cosmetic is not particularly limited as long as it has been used in a cosmetic and so forth to date and it is a hydrocarbon oil that is volatile at normal temperature (25°C). The volatile hydrocarbon oil may include isododecane, isohexadecane, hydrogenated polyisobutene and so forth.

### <(c) Volatile dimethicone>

According to the present invention, as (c) a volatile dimethicone (or hereafter simply "component c" in some cases) formulated in the sunscreen cosmetic, dimethylpolysiloxane that is less viscous and volatile at normal temperature and being used in a cosmetic to date can be used. The commercially available volatile dimethicone may include KF-96L-1.5cs and KF-96L-2cs (both from Shin-Etsu Chemical Co., Ltd.), and so forth.

The combined formulation amount of the above (b) a volatile hydrocarbon oil and (c) a volatile dimethicone should be in the range of 3 to 45% by mass, more preferably in the range of 5 to 40% by mass, most preferably in the range of 20 to 30% by mass on the basis of the total amount of the sunscreen cosmetic. When the combined formulation amount is less than 3% by mass, spreadability on the sense of application can be hardly obtained and on the other hand, when over 45% by mass, the formulation becomes greasy and the feeling-in-use can be hardly fresh.

Further, the ratio between the formulation amount of (b) a volatile hydrocarbon oil and the formulation amount of (c) a volatile dimethicone is not particularly limited, but it is in the range of 100 : 1 to 1 : 100, preferably in the range of 10 : 1 to 1 : 10, and more preferably in the range of 1 : 5 to 5 : 1.

According to the present invention, the sunscreen cosmetic may include traditionally used substances for a general cosmetic and a topical pharmaceutical agent other than the above components, e.g. a skin whitening agent, a humectant, an antioxidant, an oil component, other kind of ultraviolet light absorbers, a surfactant, a thickener, alcohols, a powder component, a colorant, an aqueous component, water, and a variety of skin nutrients can be appropriately formulated as needed.

Specifically, formulation of a semi-solid oil is preferable because of further expectable improvement on the sense of application. The semi-solid oil may include but not particularly limited to as long as it is semi-solid at normal temperature and being used for topical agents or cosmetics. For example, it may include Vaseline, hydrogenated palm oil, palm kernel oil, phytosteryl macadamiate, pentaerythrityl tetrabehenate/benzoate/ethylhexanoate, shea butter and so forth. Particularly, a plant-derived semi-solid oil (e.g. hydrogenated palm oil, palm kernel oil, phytosteryl macadamiate, shea butter and so forth) is preferable.

When a semi-solid oil is formulated, it is formulated in the range of 0.1 to 3% by mass, more preferably 0.1 to 2% by mass, even more preferably 0.3 to 2% by mass on the basis of the total amount of the sunscreen cosmetic. When the formulation amount is less than 0.1% by mass, the effectiveness of the semi-solid oil cannot be sufficiently obtained and on the other hand, when over 3% by mass, it is undesirable because stickiness therefrom may take place after application thereof.

According to the present invention, the sunscreen cosmetic can be prepared by using a traditional method used in the production of a water-in-oil emulsion composition. For example, the method includes; the oil phase except component a is heated as needed to be uniformly dissolved, and the water phase is gradually added to the oil phase, in which component a is dispersed in advance using a homogenizer, to be emulsified by using the homogenizer.

According to the present invention, the sunscreen cosmetic can be in a variety of product modes including an ointment, a cream, an emulsion, a lotion, a facial pack and so forth for any purpose.

### EXAMPLES

Further, the inventor illustrates the present invention in detail referring to Examples, but the present invention is not limited to Examples. The formulation amount is represented by % by mass (real part) unless otherwise specifically noted.

### <Evaluation method>

The method used for the evaluation of the sunscreen cosmetic of the present invention is set forth below.

### (1) Odor suppression effect

The odor threshold specific to the sunscreen cosmetic due to zinc oxide was rated in accordance with the following criteria based on the evaluation score given by 20 expert panelists.

### <Evaluation grade>

A: At least 16 panelists agreed no perceivable odor specific to the sunscreen agent.
B: 11 to 15 panelists agreed no perceivable odor specific to the sunscreen agent.
C: 6 to 10 panelists agreed no perceivable odor specific to the sunscreen agent.
D: At most 5 panelists agreed no perceivable odor specific to the sunscreen agent.

### (2) Texture

Over-all judgment of no-coarseness, good spreadability, and non-greasy sense on application and no-stickiness after application of the cosmetic, was rated in accordance with the following criteria based on the evaluation score given by 20 expert panelists.

### <Evaluation grade>

A: At least 16 panelists agreed as excellent.
B: 11 to 15 panelists agreed as excellent.
C: 6 to 10 panelists agreed as excellent.
D: At most 5 panelists agreed as excellent.

### <Testing example>

### (1) Changes of characteristics due to component a

A few general water-in-oil emulsion compositions shown in Table 1 were prepared to compare impacts on odor threshold and texture between the hydrophobized silica-coated microparticle of zinc oxide and the powder of the silicone-hydrophobized non-silica-coated microparticle of zinc oxide. Specifically, cation-modified bentonite was dispersed in an oil phase component using a homogenizer and each powder was added and dispersed using the homogenizer to obtain the oil phase, and then the water-in-oil emulsion composition was prepared by emulsifying using the homogenizer while the separately prepared water phase was gradually added to the oil phase. Each evaluation grade is shown in Table 1 together.

**[Table 1]**

| | Ingredient | Comparative Example 1 | Comparative Example 2 | Comparative Example 3 | Comparative Example 4 |
|---|---|---|---|---|---|
| Water phase | Ion-exchanged water | Balance | Balance | Balance | Balance |
| | Ethyl alcohol | 6.0 | 6.0 | 6.0 | 6.0 |
| | Glycerol | 1.0 | 1.0 | 1.0 | 1.0 |
| | Edetate trisodium | 0.1 | 0.1 | 0.1 | 0.1 |
| | Phenoxyethanol | 0.5 | 0.5 | 0.5 | 0.5 |
| Oil phase | Ethylhexyl palmitate | 7.0 | 7.0 | 7.0 | 7.0 |
| | Decamethylcyclopentasiloxane | 30.0 | 30.0 | 30.0 | 30.0 |
| | Isostearic acid | 0.5 | 0.5 | 0.5 | 0.5 |
| | Trimethylsiloxysilicate | 3.0 | 3.0 | 3.0 | 3.0 |
| | Cation-modified bentonite | 0.2 | 0.2 | 0.2 | 0.2 |
| | Octyl methoxycinnamate | 10.0 | 10.0 | 10.0 | 10.0 |
| | Polyoxyalkylene-modified polysiloxane | 1.0 | 1.0 | 1.0 | 1.0 |
| Powder part | Silicone-treated microparticle *1 of zinc oxide | 20.0 | - | - | - |
| | Silicone-treated microparticle *2 of zinc oxide | - | 20.0 | - | - |
| | (a) Silicone-treated silica-coated microparticle *3 of zinc oxide | - | - | 20.0 | - |
| | (a) Silicone-treated silica-coated microparticle *4 of zinc oxide | - | - | - | 20.0 |
| Evaluation grade | No perceivable odor specific to sunscreen | D | D | A | A |
| | No coarseness | B | B | D | D |
| | Good spreadability | B | B | D | D |

| | | | | | |
|---|---|---|---|---|---|
| *1: FINEX-50S-LP2 (manufactured by Sakai Chemical Industry Co., Ltd.) *2: MZY-505S (manufactured by Tayca Corporation) *3: FINEX-50W-LP2 (manufactured by Sakai Chemical Industry Co., Ltd.) *4: Maxlight® ZS-032-D (manufactured by Showa Denko K.K.) | | | | | |

As shown in Table 1, the strong odor specific to zinc oxide was perceived from Comparative Examples 1 and 2, which are made of a silicone-processed microparticle of zinc oxide without silica-coating. In contrast, the odor specific to zinc oxide was hardly perceived from Comparative Examples 3 and 4, which are made of a silicone-processed microparticle of zinc oxide with silica-coating. On the other hand, compared to Comparative Example 1 and 2, Comparative Example 3 and 4 provided the sense of coarseness and inferior spreadability on the application.

### (2) Changes of characteristics due to components b and c

A few water-in-oil emulsion compositions shown in Table 2 were prepared to compare impacts from the combination of (a) a hydrophobized silica-coated microparticle of zinc oxide with (b) a volatile hydrocarbon oil and (c) a volatile dimethicone, and, further a semi-solid oil in some cases. Specifically, component a is added to the oil phase components including component b and c (even semi-solid oil) and dispersed using a homogenizer to obtain the oil phase, and then the water-in-oil emulsion composition was prepared by emulsifying using the homogenizer while the separately prepared water phase was gradually added to the oil phase components. Each evaluation grade is shown in Table 2 together.

**[Table 2]**

| | Ingredient | Comparative Example 5 | Comparative Example 6 | Comparative Example 7 | Example 1 | Example 2 | Example 3 | Example 4 | Example 5 | Comparative Example 8 |
|---|---|---|---|---|---|---|---|---|---|---|
| Water phase | Ion-exchanged water | Balance | Balance | Balance | Balance | Balance | Balance | Balance | Balance | Balance |
| | Ethyl alcohol | 5 | 5 | 5 | 5 | 5 | 5 | 5 | 5 | 5 |
| | Glycerol | 5 | 5 | 5 | 5 | 5 | 5 | 5 | 5 | 5 |
| | Edetate trisodium | 0.05 | 0.05 | 0.05 | 0.05 | 0.05 | 0.05 | 0.05 | 0.05 | 0.05 |
| | Phenoxyethanol | 0.5 | 0.5 | 0.5 | 0.5 | 0.5 | 0.5 | 0.5 | 0.5 | 0.5 |
| Oil | Decamethylcyclopentasiloxane | 28 | - | - | - | - | - | - | - | - |
| phase | (b) Isododecane | - | 14 | - | 14 | 8 | 8 | 8 | 8 | 8 |
| | (b) Isohexadecane | - | 14 | - | - | 2 | 2 | 2 | 2 | 2 |
| | (c) Volatile dimethicone*5 | - | - | 14 | - | 8 | 8 | 8 | 8 | 8 |
| | (c) Volatile dimethicone*6 | - | - | 14 | 14 | 10 | 10 | 10 | 10 | 10 |
| | Phytosteryl macadamiate | - | - | - | - | - | 0.5 | 1 | 2 | 4 |
| | Diisopropyl sebacate | 5 | 5 | 5 | 5 | 5 | 5 | 5 | 5 | 5 |
| | Glyceryl 2-ethylhexanoate | 5 | 5 | 5 | 5 | 5 | 5 | 5 | 5 | 5 |
| | Polyoxyalkylene-modified polysiloxane | 3 | 3 | 3 | 3 | 3 | 3 | 3 | 3 | 3 |
| | Isostearic acid | 1 | 1 | 1 | 1 | 1 | 1 | 1 | 1 | 1 |
| | 2-Ethylhexyl p-methoxycinnamate | 5 | 5 | 5 | 5 | 5 | 5 | 5 | 5 | 5 |
| | Octocrylene | 5 | 5 | 5 | 5 | 5 | 5 | 5 | 5 | 5 |
| | Bis-ethylhexyloxyphenol methoxyphenyl triazine | 2 | 2 | 2 | 2 | 2 | 2 | 2 | 2 | 2 |
| | Diethylamino hydroxybenzoyl hexyl benzoate | 2 | 2 | 2 | 2 | 2 | 2 | 2 | 2 | 2 |
| | Dimethicodiethylbenzalmalonate | 5 | 5 | 5 | 5 | 5 | 5 | 5 | 5 | 5 |
| Powder part | Spherical polymethylsiloxane | 10 | 10 | 10 | 10 | 10 | 10 | 10 | 10 | 10 |
| | (a) Silicone-treated silica-coated microparticle *4 of zinc oxide | 5 | 5 | 5 | 5 | 5 | 5 | 5 | 5 | 5 |
| Evaluation Grade | No odor specific to sunscreen | A | A | A | A | A | A | A | A | A |
| | No coarseness | D | C | C | B | B | A | A | A | A |
| | No stickiness after application | A | A | A | A | A | A | A | A | D |

| | | | | | | | | | | |
|---|---|---|---|---|---|---|---|---|---|---|
| *4: Maxlight® ZS-032-D (manufactured by Showa Denko K.K.) *5: KF-96L-1.5cs (manufactured by Shin-Etsu Chemical Co., Ltd.) *6: KF-96L-2cs (manufactured by Shin-Etsu Chemical Co., Ltd.) | | | | | | | | | | |

As shown in Table 2, Example 1 and 2 formulating both (b) a volatile hydrocarbon oil and (c) a volatile dimethicone show better grade as for coarseness and spreadability than Comparative Example 5 to 7 in which either one or both component are not contained, and are found having excellent properties as a product.

Furthermore, coarseness is further suppressed and evaluation grades thereof are extremely excellent in all categories including odor and texture when semi-solid phytosteryl macadamiate is added (Example 3 to 5.) However, when an excess amount of phytosteryl macadamiate is formulated (Comparative Example 8), it is confirmed that texture thereof is controversially damaged because stickiness therefrom takes place after applied.

### (3) Changes of characteristics due to the total amount of component b and c

A variety of water-in-oil emulsion compositions shown in Table 3, in which only total formulation amount of (b) a volatile hydrocarbon oil and (c) a volatile dimethicone is different each other, were prepared using the same method as used for the above Example 1. Each evaluation grade is shown in Table 3 together.

**[Table 3]**

| | Ingredient | Comparative Example 9 | Example 6 | Example 7 | Example 8 | Comparative Example 10 |
|---|---|---|---|---|---|---|
| Water phase | Ion-exchanged water | Balance | Balance | Balance | Balance | Balance |
| | Ethyl alcohol | 5 | 5 | 5 | 5 | 5 |
| | Glycerol | 2 | 2 | 2 | 2 | 2 |
| | Edetate trisodium | 0.05 | 0.05 | 0.05 | 0.05 | 0.05 |
| | Phenoxyethanol | 0.5 | 0.5 | 0.5 | 0.5 | 0.5 |
| Oil phase | Decamethylcyclopentasiloxane | 20 | 20 | - | - | - |
| | (b) Isododecane | 1 | 2.5 | 14 | 20 | 25 |
| | (c) Volatile dimethicone*6 | 1 | 2.5 | 14 | 20 | 25 |
| | Phytosteryl macadamiate | 0.5 | 0.5 | 0.5 | 0.5 | 0.5 |
| | Diisopropyl sebacate | 5 | 5 | 5 | 5 | 5 |
| | Glyceryl 2-ethylhexanoate | 5 | 5 | 5 | 5 | 5 |
| | Polyoxyalkylene-modified polysiloxane | 3 | 3 | 3 | 3 | 3 |
| | Isostearic acid | 1 | 1 | 1 | 1 | 1 |
| | 2-Ethylhexyl p-methoxycinnamate | 5 | 5 | 5 | 5 | 5 |
| | Bis-ethylhexyloxyphenol methoxyphenyl triazine | 2 | 2 | 2 | 2 | 2 |
| | Diethylamino hydroxybenzoyl hexyl benzoate | 2 | 2 | 2 | 2 | 2 |
| Powder part | Spherical polymethylsiloxane | 5 | 5 | 5 | 5 | 5 |
| | (a) Silicone-treated silica-coated microparticle *4 of zinc oxide | 10 | 10 | 10 | 10 | 10 |
| Evaluation grade | No odor specific to sunscreen | A | A | A | A | A |
| | No coarseness | C | B | A | A | A |
| | No greasiness | B | B | A | A | C |
| | No stickiness after application | A | A | A | A | A |

| | | | | | | |
|---|---|---|---|---|---|---|
| *4: Maxlight® ZS-032-D (manufactured by Showa Denko K.K.) *6: KF-96L-2cs (manufactured by Shin-Etsu Chemical Co., Ltd.) | | | | | | |

As shown in Table 3, when the total amount of (b) a volatile hydrocarbon oil and (c) a volatile dimethicone is less than 3% by mass, spreadability worsens and coarse feeling takes place on application and when the total amount is over 45% by mass, the greasiness likely takes place (Comparative Example 9 and 10.) In contrast, it is confirmed that Examples 6 to 8, in which the total amount of component b and c is in the range of 3 to 45% by mass, provides an excellent texture with no coarseness and no greasiness.

### (4) Changes in characteristics due to a semi-solid oil

Each water-in-oil emulsion composition containing a different kind of semi-sold oil but the same amount (1% by mass) was prepared using the same method as used for the above Example 1. Each evaluation grade is shown in Table 4 together.

**[Table 4]**

| | Ingredient | Example 9 | Example 10 | Example 11 |
|---|---|---|---|---|
| Water phase | Ion-exchanged water | Balance | Balance | Balance |
| | Ethyl alcohol | 5 | 5 | 5 |
| | Glycerol | 5 | 5 | 5 |
| | Edetate trisodium | 0.05 | 0.05 | 0.05 |
| | Phenoxyethanol | 0.5 | 0.5 | 0.5 |
| Oil phase | Decamethylcyclopentasiloxane | - | - | - |
| | (b) Isododecane | 8 | 8 | 8 |
| | (b) Isohexadecane | 2 | 2 | 2 |
| | (c) Volatile dimethicone*5 | 8 | 8 | 8 |
| | (c) Volatile dimethicone*6 | 10 | 10 | 10 |
| | Phytosteryl macadamiate | 1 | - | - |
| | Hydrogenated palm oil | - | 1 | - |
| | Shea butter | - | - | 1 |
| | Diisopropyl sebacate | 5 | 5 | 5 |
| | Glyceryl 2-ethylhexanoate | 5 | 5 | 5 |
| | Polyoxyalkylene-modified polysiloxane | 3 | 3 | 3 |
| | Isostearic acid | 1 | 1 | 1 |
| | 2-Ethylhexyl p-methoxycinnamate | 5 | 5 | 5 |
| | Octocrylene | 5 | 5 | 5 |
| | Bis-ethylhexyloxyphenol methoxyphenyl triazine | 2 | 2 | 2 |
| | Diethylamino hydroxybenzoyl hexyl benzoate | 2 | 2 | 2 |
| | Dimethicodiethylbenzalmalonate | 5 | 5 | 5 |
| Powder part | Spherical polymethylsiloxane | 10 | 10 | 10 |
| | (a) Silicone-treated silica-coated microparticle *4 of zinc oxide | 5 | 5 | 5 |
| Evaluation grade | No odor specific to sunscreen | A | A | A |
| | No coarseness | A | A | A |
| | No stickiness after application | A | A | A |

| | | | | |
|---|---|---|---|---|
| *4: Maxlight® ZS-032-D (manufactured by Showa Denko K.K.) *5: KF-96L-1.5cs (manufactured by Shin-Etsu Chemical Co., Ltd.) *6: KF-96L-2cs (manufactured by Shin-Etsu Chemical Co., Ltd.) | | | | |

As shown in Table 4, when any semi-solid oils including phytosteryl macadamiate, hydrogenated palm oil and shea butter are employed, evaluation grades are extremely excellent in all categories including odor and texture (Example 9 to 11.)

| Formulation Example 1: Sunscreen emulsion | % by mass |
|---|---|
| Isododecane | 10 |
| Isohexadecane | 4 |
| Volatile dimethicone | 18 |
| Hydrogenated palm oil | 0.3 |
| Squalane | 2 |
| Pentaerythrityl tetraethylhexanoate | 5 |
| Isopropyl myristate | 5 |
| Polyoxyalkylene-modified polysiloxane | 2 |
| Bis-ethylhexyloxyphenol methoxyphenyl triazine | 2 |
| Diethylamino hydroxybenzoyl hexyl benzoate | 2 |
| 2-Ethylhexyl p-methoxycinnamate | 10 |
| Spherical silica powder | 10 |
| Hydrophobized silica-coated microparticle of zinc oxide | 5 |
| Ethyl alcohol | 5 |
| 1,3-Butylene glycol | 5 |
| Phenoxyethanol | 0.5 |
| Fragrance | q. s. |
| Ion-exchanged water | Balance |

| Formulation Example 2: Sunscreen emulsion | % by mass |
|---|---|
| Decamethylcyclopentasiloxane | 1 |
| Isododecane | 5 |
| Isohexadecane | 5 |
| Volatile dimethicone | 8 |
| Non-volatile dimethicone | 3 |
| Phytosteryl macadamiate | 0.5 |
| Cetyl 2-ethylhexanoate | 5 |
| Pentaerythrityl tetraethylhexanoate | 5 |
| Diisopropyl sebacate | 8 |
| Glyceryl 2-ethylhexanoate | 8 |
| Polyoxyalkylene-modified polysiloxane | 3 |
| Octocrylene | 5 |
| Dimethicodiethylbenzalmalonate | 3 |
| 4-t-Butyl-4'-methoxydibenzoylmethane | 1 |
| Methylene bis-benzotriazolyl tetramethylbutylphenol | 2 |
| Spherical nylon powder | 6 |
| Hydrophobized silica-coated microparticle of zinc oxide | 10 |
| 1,3-Butylene glycol | 5 |
| Phenoxyethanol | 0.5 |
| Edetate trisodium | 0.05 |
| Fragrance | q. s. |
| Ion-exchanged water | Balance |

| Formulation Example 3: Sunscreen emulsion | % by mass |
|---|---|
| Isohexadecane | 17 |
| Volatile dimethicone | 17 |
| Non-volatile dimethicone | 2 |
| Phytosteryl macadamiate | 1 |
| Squalane | 2 |
| Pentaerythrityl tetraethylhexanoate | 3 |
| Diisopropyl sebacate | 3 |
| Isopropyl myristate | 3 |
| Polyoxyalkylene-modified polysiloxane | 2 |
| Bis-ethylhexyloxyphenol methoxyphenyl triazine | 2 |
| Diethylamino hydroxybenzoyl hexyl benzoate | 2 |
| 2-Ethylhexyl p-methoxycinnamate | 10 |
| Spherical PMMA powder | 6 |
| Hydrophobized silica-coated microparticle of zinc oxide | 13 |
| Ethyl alcohol | 2 |
| Glycerol | 3 |
| Paraben | 0.5 |
| Fragrance | q. s. |
| Ion-exchanged water | Balance |

| Formulation Example 4: Sunscreen cream | % by mass |
|---|---|
| Cation-modified bentonite | 2 |
| Isododecane | 20 |
| Volatile dimethicone | 10 |
| Hydrogenated palm oil | 0.5 |
| Glyceryl 2-ethylhexanoate | 5 |
| Isopropyl myristate | 5 |
| Polyoxyalkylene-modified polysiloxane | 2 |
| Octocrylene | 3 |
| Bis-ethylhexyloxyphenol methoxyphenyl triazine | 1 |
| 2-Ethylhexyl p-methoxycinnamate | 5 |
| Phenylbenzimidazole sulfonic acid | 1 |
| Spherical silicone resin powder | 6 |
| Hydrophobized silica-coated microparticle of zinc oxide | 4 |
| Hydrophobized microparticle of titanium dioxide | 2 |
| Dipropylene glycol | 5 |
| Glycerol | 3 |
| Edetate trisodium | 0.1 |
| Phenoxyethanol | 0.5 |
| Fragrance | q. s. |
| Neutralizer | q. s. |
| Ion-exchanged water | Balance |

## Claims

1. A water-in-oil emulsion sunscreen cosmetic comprising;
(a) a hydrophobized silica-coated microparticle of zinc oxide,
(b) a volatile hydrocarbon oil,
(c) a volatile dimethicone, and
(d) optionally a semi-solid oil formulated in an amount of 3% by mass or less on the basis of the total amount of the sunscreen cosmetic, and
wherein the total amount of (b) the volatile hydrocarbon oil and (c) the volatile dimethicone is in the range of 3 to 45% by mass on the basis of the total amount of the sunscreen cosmetic.

2. The water-in-oil emulsion sunscreen cosmetic according to claim 1,
wherein an amount of the semi-solid oil formulated is 0.1 to 3% by mass on the basis of the total amount of the sunscreen cosmetic.

## Patentansprüche

1. Sonnenschutzkosmetikum in Form einer Wasser-in-Öl-Emulsion, umfassend:
(a) ein mit hydrophobiertem Silica beschichtetes Mikroteilchen aus Zinkoxid,
(b) ein flüchtiges Kohlenwasserstofföl,
(c) ein flüchtiges Dimethicon, und
(d) wahlweise ein halbfestes Öl, formuliert in eine Menge von 3 Masse-% oder weniger auf der Basis der gesamten Menge des Sonnenschutzkosmetikums, und
wobei die Gesamtmenge an (b) dem flüchtigen Kohlenwasserstofföl und (c) dem flüchtigen Dimethicon im Bereich von 3 bis 45 Masse-% liegt auf der Basis der gesamten Menge des Sonnenschutzkosmetikums.

2. Sonnenschutzkosmetikum in Form einer Wasser-in-Öl-Emulsion nach Anspruch 1, wobei eine Menge des formulierten halbfesten Öls 0,1 bis 3 Masse-% beträgt auf der Basis der gesamten Menge des Sonnenschutzkosmetikums.

## Revendications

1. Un produit cosmétique de protection solaire en émulsion eau dans l'huile comprenant :
(a) une microparticule d'oxyde de zinc revêtue de silice rendu hydrophobe,
(b) une huile hydrocarbonée volatile, et
(c) un diméthicone volatile, et
(d) facultativement une huile semi-solide, dans lequel une quantité de l'huile semi-solide formulée est de 3% en masse ou inférieur sur la base de la quantité totale du produit cosmétique de protection solaire,
dans lequel la quantité totale de (b) l'huile volatile hydrocarbonée et (c) du diméthicone volatile est dans la gamme de 3 à 45% en masse sur la base de la quantité totale du produit cosmétique de protection solaire.

2. Le produit cosmétique de protection solaire en émulsion eau dans l'huile selon la revendication 1,
dans lequel une quantité de l'huile semi-solide formulée est de 0,1 à 3% en masse sur la base de la quantité totale du produit cosmétique de protection solaire.
